(19) Europäisches Patentamt
European Patent Office
Office européen des brevets

(11) Veröffentlichungsnummer: **0 325 115 A2**

(12) **EUROPÄISCHE PATENTANMELDUNG**

(21) Anmeldenummer: **89100123.2**

(22) Anmeldetag: **05.01.89**

(51) Int. Cl.4: **C07C 127/19 , C08G 18/78 , C08G 18/46**

(30) Priorität: **16.01.88 DE 3801091**

(43) Veröffentlichungstag der Anmeldung: **26.07.89 Patentblatt 89/30**

(84) Benannte Vertragsstaaten: **AT BE DE ES FR GB IT NL SE**

(71) Anmelder: **BAYER AG**

**D-5090 Leverkusen 1 Bayerwerk(DE)**

(72) Erfinder: **Ruckes, Andreas, Dr.**
**Herderstrasse 13**
**D-5090 Leverkusen 3(DE)**
Erfinder: **Grögler, Gerhard, Dr.**
**Von-Diergardt-Strasse 48**
**D-5090 Leverkusen(DE)**
Erfinder: **Kopp, Richard, Dr.**
**Bilharzstrasse 15**
**D-5000 Koeln 80(DE)**
Erfinder: **Hess, Heinrich, Dr.**
**Körnerstrasse 5**
**D-5090 Leverkusen(DE)**

(54) **N,N'-Bis (5-isocyanatonaphthyl) harnstoff, Verfahren zu dessen Herstellung und Verwendung.**

(57) Die vorliegende Erfindung betrifft N,N′-(5-isocyanatonaphthyl)harnstoff, erhältlich aus der Umsetzung von Naphthalin-1,5-diisocyanat (NDI) und Wasser in einem inerten lösungsmittel. Der erfindungsgemäße Harnstoff, der gegebenenfalls einen kleinen Anteil an oligomeren Harnstoffen enthält, läßt sich vorteilhaft beim Aufbau von Polyurethan- oder Polyharnstoff-Elastomeren ver wendungen.

EP 0 325 115 A2

## N,N′-Bis(5-isocyanatonaphthyl)harnstoff, Verfahren zu dessen Herstellung und Verwendung

Die vorliegende Erfindung betrifft N,N′-Bis-(5-isocyanatonaphthyl)harnstoff, erhältlich aus der Umsetzung von Naphthalin-1,5-diisocyanat (NDI) und Wasser in einem inerten Lösungsmittel. Der erfindungsgemäße Harnstoff, der gegebenenfalls einen kleinen Anteil an oligomeren Harnstoffen enthält, läßt sich vorteilhaft beim Aufbau von Polyurethan- oder Polyharnstoff-Elastomeren verwenden.

Es ist bekannt, daß Monoisocyanate mit Wasser unter Bildung N-substituierter Carbamidsäurederivate reagieren, die under Kohlendioxidabspaltung und Reaktion mit weiterem Isocyanat in Harnstoffderivate übergehen. Analog erhält man aus Diisocyanaten und Wasser durch Polyaddition Polyharnstoffe.

Es ist ferner bekannt, daß man bei Einhaltung bestimmter Reaktionsbedingungen niedermolekulare Harnstoffdiisocyanate der allgemeinen Formel

$$OCN-R-NH-\overset{O}{\overset{\|}{C}}-NH-R-NCO$$

isolieren kann, wenn man 1 Mol Wasser mit mindestens 2 Mol eines Diisocyanates bei niedrigen Temperaturen zwischen 0°C und 30°C in einem Lösungsmittel reagieren läßt. Wie in der DAS-1 101 394 beschrieben, sind für diese Reaktion nur solche Diisocyanate geeignet, die wie z.B. das 2,4-Toluylendiisocyanat Isocyanatgruppen mit verschieden großer Reaktionsfreudigkeit aufweisen.

In der US-PS-2 757 185 wird gelehrt, daß Verbindungen wie meta- oder para-Phenylendiisocyanat bei der Reaktion mit Wasser keine monomeren, sondern nur polymere Harnstoffe bilden.

Überraschenderweise wurde nun gefunden, daß man N,N′-Bis(5-isocyanatenaphthyl)harnstoff, der gegebenenfalls nur einen kleinen Anteil an oligomeren Harnstoffen enthält, in guter Ausbeute erhält, wenn man Naphthalindiisocyanat in inerten Lösungsmitteln, in einer Konzentration ≥ 2 Gew.-%, bezogen auf das verwendete Lösungsmittel, mit Wasser im Molverhältnis 2:1 umsetzt.

Gegebenenfalls kann ein basischer Katalysator mitverwendet werden.

Der erfindungsgemäße Harnstoff läßt sich mit Vorteil zur Herstellung von Polyurethan- oder Polyharnstoff-Elastomeren verwenden.

Im Vergleich zu auf NDI basierenden Elastomeren findet man neben ausgezeichnete mechanische Eigenschaften vor allem einen sehr hohen Wärmestand.

Aufgrund seines hohen Schmelzpunktes eignet sich der erfindungsgemäße Harnstoff auch als heterogene Isocyanatkomponente in Einkomponenten-Systemen nach der DE-OS-3 230 757.

Ein weiterer Vorteil besteht darin, daß die Sublimationsneigung des NDI's, die sich bei dessen Verarbeitung in der Schmelze als störend erweist, beim entsprechenden Harnstoff nicht auftritt, wodurch eine einfache und sichere Handhabung gewährleistet ist.

Gegenstand der vorliegenden Erfindung ist somit der N,N′-(5-isocyanatonaphthyl)harnstoff I, der gegebenenfalls einen kleinen Anteil an oligomeren Harnstoff enthält, erhältlich auch der Umsetzung von

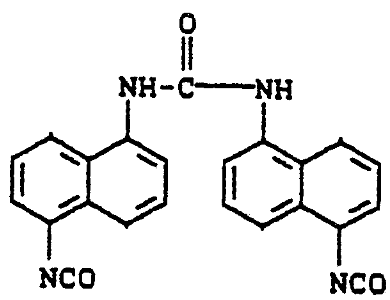

I,

Naphtalin-1,5-diisocyanat und Wasser unter den im folgenden näher beschriebenen Bedingungen.

Als Ausgangskomponente für das erfindungsgemäße Verfahren ist z.B. das von der Bayer AG unter dem Handelsnamen Desmodur 15® erhältliche Naphthalin-1,5-diisocyanat geeignet.

Als Lösungsmittel werden solche verwendet, in dem das NDI und wenigstens ein Teil des benötigten Wassers löslich sind. Es soll weiterhin keine NCO-reaktiven Gruppen enthalten, also inert oder nicht-reaktiv gegenüber Isocyanaten sein.

Ether, wie Dioxan oder Diisopropylether, Ketone, wie Aceton, Methylethylketon und Ester, wie Isopropylacetat oder Cellosolveacetat stellen geeignete Lösungsmittel dar. Halogenkohlenwasserstoffe, in denen Wasser praktisch unlöslich ist, sind z.B. ungeeignet als erfindungsgemäße Lösungsmittel. Es ist jedoch nicht notwendig, daß sich die gesamte benötigte Menge Wasser im verwendeten Lösungsmittel löst.

Bevorzugte Lösungsmittel sind Dioxan und Diisopropylether.

Die Konzentration des Naphthalindiisocyanats im Lösungsmittel ist im allgemeinen unkritisch. Im Falle zu großer Verdünnung wird jedoch der Harnstoff unter Umständen mit stark erniedrigtem NCO-Gehalt erhalten.

Bei zu stark konzentrierten Lösungen besteht andererseits die Gefahr, daß das ausfallende Reaktionsprodukt mit unreagiertem NDI verunreinigt ist.

Bevorzugt wird eine Konzentration von ≧ 2 Gew.-% NDI, bezogen auf das Lösungsmittel eingehalten.

Die Menge an Wasser entspricht in etwa der theoretisch benötigten Menge zur Reaktion mit zwei Isocyanat-Gruppen. Wird zu wenig Wasser verwendet, bleibt unreagiertes Ausgangsisocyanat zurück, welches abgetrennt werden muß. Andererseits wird bei Verwendung von zuviel Wasser nur ein Polyharnstoff mit stark erniedrigtem NCO-Gehalt erhalten.

Der entstehende Harnstoff ist in den oben erwähnten Lösungsmitteln nahezu unlöslich und entzieht sich so einer Weiterreaktion der noch vorhandenen NCO-Gruppen mit Wasser, so daß im allgemeinen von dem eingesetzten NDI nur eine NCO-Gruppe pro Molekül an der Harnstoffgruppenbildung teilnimmt.

Zur Erzielung besserer Raum-Zeit-Ausbeuten kann zur Beschleunigung der Reaktion zwischen Wasser und NDI bei erhöhter Temperatur (vorzugsweise RT -110° C) gearbeitet und/oder ein Katalysator mitverwendet werden. Zur Anwendung kommen bevorzugt die in der Polyurethanchemie üblicherweise eingesetzten Katalysatoren, wie z.B. tertiäre Amine (N,N-Dimethylbenzylamin) oder metallorganische Verbindungen (Zinn(II)dioctoat, Dibutylzinndiacetat).

Nach Beendigung der Reaktion wird das ausgefallende Produkt über ein geeignetes Filter abgesaugt und mit einem inerten Lösungsmittel, wie z.B. oder Petrolether gewaschen und in einem Trockenschrank, vorzugsweise in Vakuum, bei niedrigen Temperaturen getrocknet.

Der NCO-Gehalt des nach diesem Verfahren erhaltenen Produktes liegt normalerweise nur geringfügig unterhalb des berechneten NCO-Gehaltes. Um befriedigende Raum-Zeit-Ausbeuten zu erhalten, müssen jedoch die Reaktionsbedingungen im allgemeinen so gewählt werden, daß Produkte mit einem gegenüber der Theorie verringertem NCO-Gehalt, bei denen also geringfügige Oligoharnstoffbildung eingetreten ist, erhalten werden. Es zeigt sich nämlich, daß alle Maßnahmen, die zu einer Erhöhung der Reaktionsgeschwindigkeit führen, wie erhöhte Temperatur, polare, wasserlösliche Lösungsmittel, zwar eine Steigerung der Ausbeute bei verringerter Reaktionszeit, aber gleichzeitig immer eine Verringerung des NCO-Gehaltes des erfindungsgemäßen Harnstoffes bewirken. Die Produkte lassen sich jedoch verwenden, wenn die Oligoharnstoffbildung bestimmte Grenzen nichtüberschreitet.

Das lagerstabile und leicht handhabbare Produkt fällt in Form eines feinteiligen, kristallinen Pulvers an, das, abhängig von den Reaktionsbedingungen, einen NCO-Gehalt von mindestens 16 Gew.-%, vorzugsweise mindestens 19 Gew.-% aufweist.

Der erfindungsgemäße Harstoff läßt sich in seiner niedermolekularen bzw. niederoligomeren Form mit Vorteil zum Aufbau von Polyurethansystemen verwendet. Er wird im allgemeinen zunächst fein gemahlen, was z.B. in einer Kugelmühle geschehen kann, bis er eine durchschnittliche Korngröße von 1 bis 50 µm, vorzugsweise 3 bis 10 µm, besitzen.

Eine bevorzugte Anwendung beinhaltet die Herstellung von Gießelastomeren, in denen als NCO-reaktive Komponenten Polyesterpolyole bzw. mit aromatischen Amingruppen terminierte Polyester eingesetzt werden. Die Polyesterpolyamine werden vorzugsweise nach der E-AS O 219 035 durch Hydrolyse von endständigen Isocyanatgruppen aufweisende Verbindungen erhalten. Bei diesem Verfahren werden insbesondere zwei oder drei Hydroxylgruppen aufweisende Polyester zu NCO-Prepolymeren umgesetzt und in einem zweiten Schritt die Isocyanatgruppen durch Hydrolyse in eine Aminogruppe überführt. Man erhält Elastomere mit hervorragendem Wärmestand und ausgezeichneten mechanischen Eigenschaften.

Die folgenden Beispiele erläutern die Herstellung des erfindungsgemäßen Harnstoffes und seine Anwendung zur Herstellung von hochwertigen Polyurethanelastomeren.

In den Beispielen wird als Napthalindiisocyanat-1,5 das von der Bayer AG als Desmodur 15® erhältliche Produkt eingesetzt.

Beispiele

Beispiel 1

Herstellung des erfindungsgemäßen Harnstoffes

In 4 l Dioxan werden 287 g (1,36 Mol) Desmodur 15® gelöst. Nach Zugabe von 12,5 ml (0.69 Mol) $H_2O$ wird die Reaktionsmischung 20 h bei Raumtemperatur gerührt. Das ausgefallene produkt wird abfiltriert, mit Dioxan gewaschen und im Vakuumtrockenschrank getrocknet. Man erhält 52 g eines NDI-Harnstoffes mit einem NCO-Gehalt von 20.0 Gew.-%. Aus dem Filtrat können nach insgesamt 6-tägigem Stehen bei Raumtemperatur weiter 122 g Produkt mit einem NCO-Gehalt von 21.0 Gew.-% gewonnen werden. Insgesamt erhält man den NDI-Harnstoff in einer Ausbeute von 65 %, bezogen auf eingesetztes Desmodur 15, mit einem durchschnittlichen NCO-Gehalt von 20.7 Gew.-% (ber.: 21.3 Gew.-%).

Beispiel 2

Herstellung eines Gießelastomeren mit einem Polyester

200 g eines linearen Polyesters aus Adipinsäure und Ethylenglykol (MG:2000, OH-Zahl:56 mg KOH/g) werden bei 80 - 100 °C kurze Zeit im Wasserstrahlvakuum entwässert. Nach dem Abkühlen auf 50 - 60 °C setzt man der Schmelze 0,3 g Octa-Soligen Pb (Pb-octoat) zu. Anschließend werden mittels eines geeigneten Rühraggregates 48.6 g NDI-Harnstoffdiisocyanat aus Beispiel 1 (NCO-Gehalt: 20.7 Gew.-%) in Form eines geingemahlenen Pulvers (Teilchengröße: 10 - 50 µm) eingerührt. Bei dieser Temperatur erfolgt noch keine Reaktion zwischen dem Polyester und dem NDI-Harnstoff-diisocyanat. Nach kurzem Entgasen kann der flüssige Reaktionsansatz in eine mit Trennmittel versehene und vorgewärmte Form gegossen werden. Diese wird dann 3 - 4 h auf 140 - 150 °C erwärmt, man erhält eine hochelastischen Formkörper mit den in Tabelle 1 angegebenen mechanischen Eigenschaften.

Beispiel 3

Herstellung eines Gießelastomeren mit einem Polyesteramin

In diesem Beispiel wird ein Polyesteramin verwendet, das gemäß EP-AS-O 219 035 durch basische Hydrolyse eines NCO-Prepolymeren aus 1 Mol des in Beispiel 2 verwendeten Polyester und 2 Mol 2,4-Diisocyanatotoluol mit einer NH-Zahl von 44,3 mg KOH/g hergestellt wurde. In ähnlicher Arbeitsweise wie in Beispiel 1 beschrieben, werden der Schmelze des Polyesteramins 38.5 g NDI-Harnstoff-Diisocyanat eingerührt. Die Mitverwendung von Pb-octoat ist nicht erforderlich. Nach dem Entgasen wird das Reaktionsgemisch in eine entsprechende Form gegossen und dann 3 - 4 h auf 140 - 150 °C erhitzt. Das erhaltene hochwertige PUR-Elastomer besitzt - wie auch das in Beispiel 2 beschriebene eine überraschend hohe Wärmebeständigkeit. In Tabelle 1 sind dazu die mechanischen Eigenschaften angegeben.

Tabelle 1

| Mechanische Eigenschaften der Formkörper nach Beispiel 2 und 3 | | |
|---|---|---|
| | Beispiel 1 | Beispiel 2 |
| Zugfestigkeit (DIN53504) | 21.0 MPa | 29.5 MPa |
| Bruchdehnung (DIN53504) | 700 % | 550 % |
| Weiterreißwiderstand (DIN53515) | 65 KN/m | 97 KN/m |
| Shore-Härte A (DIN53505) | 94 | 97 |
| Elastizität (DIN53512) | 48% | 45 % |

## Ansprüche

1. N,N′-Bis(5-isocyanatonaphthyl)harnstoff
2. N,N′-Bis(5-isocyanatonaphthyl)harnstoff mit einem NCO-Gehalt von mindestens 17 Gew.-%.

3. Verfahren zur Herstellung von Harnstoffen gemäß den Ansprüchen 1 und 2, dadurch gekennzeichnet, daß man Naphthalin-1,5-diisocyanat mit äquimolaren Wassermengen in einem inerten Lösungsmittel hydrolysiert.

4. Verwendung der Harnstoffe nach den Ansprüchen 1 oder 2 als Reaktionskomponente von NCO-reaktiven Verbindungen zur Herstellung von Polyurethanen.

5. Verwendung nach Anspruch 4, dadurch gekennzeichnet, daß als NCO-reaktive Verbindungen vorzugsweise Polyesterpolyole oder mit aromatischen Aminogruppen terminierte Polyester eingesetzt werden.

6. Verwendung nach Anspruch 5, dadurch gekennzeichnet, daß die Aminopolyester durch Hydrolyse von endständige Isocyanatgruppen aufweisenden Polyesterprepolymeren erhalten werden.